# EUROPEAN PATENT APPLICATION

(11) **EP 4 470 372 A1**
(43) Date of publication of application: **04.12.2024**
(21) Application number: 23176246.9
(22) Date of filing: 30.05.2023
(51) Int. Cl.: A01K 67/033

(54) **MOBILE WORKSTATION FOR SOILLESS CULTIVATION IN AUTOMATED INSECT FARMING**

(71) Applicant: Nasekomo B.V., 1075 HP Amsterdam (NL)
(72) Inventor: Bolard, Marc, 1407 Sofia (BG)
(74) Representative: Fidal Innovation

(57) **Abstract**

A mobile workstation (10) for an automated insect farming installation (1) comprises a displacement system (23, 24) to move the mobile workstation (10) by mechanical cooperation with a guiding system (22) of the insect farming installation (1), and a control system (11) to control the mobile workstation (10). The mobile workstation (10) is alimented by one source of energy (43) and comprises one active system to perform one action onto a substrate. The active system comprises a ploughing system (40). The mobile workstation (10) comprises an actuation system (30) to place the ploughing system (40) alternatively in an active condition where it is adapted to plough the substrate and a passive condition where it is adapted to stay outside of the substrate.

## Description

### FIELD OF THE INVENTION

The present invention relates to a mobile workstation for soilless cultivation used in an installation to effectively breed insects by the digestion of organic matter.

### BACKGROUND OF THE INVENTION

Some insects efficiently convert organic matter into protein, which can be used for animal feeding. One solution to recycle available organic matter is to set up a biological treatment by replicating at a large scale the natural process of organic matter conversion.

A whole farming cycle is made of 4 steps:
- Step 1: Organic streams are collected from agricultural or food and beverage co-products for example, like breweries, ethanol plants and/or others.
- Step 2: Insects are reared in a highly bio-secure environment and fed with the organic co-products.
- Step 3: Insects are collected from the remaining frass.
- Step 4: On one hand, insects are dried, and a protein meal and a fatty fraction are separated and further processed into animal feed ingredients or other products.
- Step 5: On the other hand, the insect frass is extracted to be used as organic fertilizer for agricultural plantation, biogas production, etc..

Thus, the organic fertilizer is useful for vegetable farming, and the protein-rich meal and the insect oil are produced to feed animals.

Such process makes it possible to artificially reproduce the complete natural cycle of organic matter. Some pollution and waste of resources are avoided by replicating the whole natural cycle thanks to insect breeding.

Among the advantages of such process, the environmental impact is highly reduced, in terms of water consumption, agricultural land usage, feed consumption.

The above step 2 can be divided into two phases: A first phase comprises a maturation of insect eggs until the larvae hatch and grow for a few days, for example five days. A second aging phase in a substrate lasts for 4 to 15 days, for example 7 to 8 days, and results in mature larvae and frass. The present invention may be used during this second aging phase.

An installation comprising generally containers and a multifunctional equipment to effectively breed insects by the digestion of organic matter is needed to ensure an efficient industrializing of this second aging phase.

The document CN 110432229 A describes such a multifunctional equipment which combines several elements. This equipment comprises an on-board motor connected to a displacement mechanism and a substrate homogenization mechanism. This equipment further comprises a motorized discharge scraper mechanism adapted to push the substrate out of the rearing container.

However, the height of the multifunctional equipment is not optimized, especially due to the insect seeder mechanism. Further, frequent actions by operators directly on the substrate are still required, at least for substrate monitoring and humidifying. Thus, the height between two horizontal work areas has to remain sufficient for the seeder mechanism and to let the operator access the substrate to perform some operations on the substrate. So, the prior art equipment is bulky and does not perform all the actions.

The invention thus aims at proposing a mobile workstation to reduce the vertical space requested between two containers and to optimize the breeding of the insects by allowing the culture of the larvae over the whole thickness of the substrate. The thickness of the substrate can also be increased compared to the prior art.

### BRIEF SUMMARY OF THE INVENTION

Thus, the invention relates to a mobile workstation for automated insect farming installation, comprising a displacement system to move the mobile workstation by mechanical cooperation with a guiding system of the insect farming installation, a control system adapted to control the mobile workstation, said mobile workstation being alimented by at least one source of energy and comprising at least one active system adapted to perform one action onto a substrate, wherein the active system comprises a ploughing system, characterized in that the mobile workstation comprises an actuation system adapted to place the ploughing system alternatively in an active condition where it is adapted to plough the substrate and a passive condition where it is adapted to stay outside of the substrate.

Upstream of the workstation, a robotic seeding installation integrates insect larvae into a substrate which also provides the nutrients, for example coming from an organic biomass stream.

The multifunctional workstation is dedicated to be used in an automated farming installation comprising a plurality of parallel levels, wherein each level comprises at least a guiding element to guide the workstation above the substrate laid in a longitudinal continuous container. When several longitudinal continuous containers are installed one above the other, a lift system to move the workstation from one container to another container can also be part of the automated farming installation.

Thanks to these provisions, the bulk of the workstation is reduced. The ratio of the volume of substrate to the total volume of the installation is increased. The versatility of the installation is improved, because the ploughing system will be deployed only when necessary.

According to the invention, the substrate may comprise a mixture of non-nutritive fibers and nutriments which, itself, is mixed with insect larvae. As insect larvae consume the nutrients, they produce frass, so that, at some point in time, the substrate comprises a mixture of non-nutritive fibers, nutriments and frass which, itself, is mixed with insect larvae. Nutriments may comprise organic biomass, seeds, plant sprouts and/or fungi.

The substrate is for example a mix of insect larvae with nutrients, such as for example organic biomass as collected from agricultural co-products, like in ethanol plants or breweries.

According to an example, the substrate is made of 4- or 5- days old larvae and organic biomass at the beginning of the second aging phase. At the end of the second aging phase, the substrate mainly comprises mature insects and frass. Some unprocessed organic biomass remains. During the second aging phase, some actions have to be performed to achieve the substrate transformation.

An automated farming installation can be extended horizontally, with the only limit of the speed of the mobile workstation combined with the number of actions to be performed over a limited period of time.

According to the invention, the mobile workstation is movable over a portion of the work surface to perform at least one sub-action controlled by the controller.

Repeating several sub-actions means that one main action (loading, applying one of the treatments, unloading, ...) is performed by the mobile workstation only on a portion of a work surface.

Depending on the action, the portion of the work surface that can be treated can be longer or shorter. To complete one action, sub-actions are repeated over different work surfaces by the same mobile workstation. Sub-actions of one main action can be performed over different portions of product. Sub-actions of one action are identical. In some cases, the portion of product to be treated is reduced to a sub-portion of the largest possible portion for one given action in order to target the corresponding work surface. In some other cases, one sub-action can be repeated on the same portion of product.

According to some embodiments, the automated farming installation further comprises at least one longitudinal guiding rail parallel to the longitudinal continuous container and guiding a movement of the mobile workstation over the work surface.

According to some embodiments, the automated farming installation further comprises a lift system comprising:
. A static structure,
. A vertically movable part, vertically movable with respect to the static structure, and adapted to receive, to support and to deliver the mobile workstation.

According to some embodiments, the lift system further comprises a second vertically movable part, vertically movable with respect to the static structure.

According to some embodiments, the second vertically movable part comprises a conveyor belt actuatable to pour a load of substrate on the work surface.

According to some embodiments, the second vertically movable part comprises a base structure and a mobile carrier movable with respect to the base structure to an expanded position where it extends outside of the static structure to either receive or deliver substrate.

According to some embodiments, the lift system comprises an engine and a counterweight and comprises a switching system alternately assembling and disassembling the first and the second vertically movable part to/from one another.

The counterweight is fixed on the other extremity to balance the weight of the vertically movable part, the mobile workstation, the conveyor and one load of product.

According to some embodiments, the automated farming installation further comprises a load relief system adapted to alternately assemble the vertically movable part to the static structure and disassemble it therefrom.

According to some embodiments, the vertically movable part comprises a base structure and a mobile workstation holder, and the mobile workstation holder is movable with respect to the base structure within the vertically movable part.

The movable workstation has a limit in terms of weight that it can unload. So, the portion of the work surface that can be unloaded of substrate is limited by the weight/volume of substrate the mobile workstation can move. Thus, to fully unload the automated farming installation with substrate, if the substrate weight is greater than the weight movable capacity of the mobile workstation, the unloading action is split into several unloading sub-action. Each of these sub-actions can be performed on a different portion of the work surface.

Thus, the substrate is brought to the next treatment phase of the whole cycle: sorting, or drying, or any other treatment phase requested after the second aging phase.

Thus, automatic insect culture is possible with very limited man action.

The portion of the work surface that can be loaded with one product is limited by the weight/volume that the mobile workstation can move. Thus, to fully load the automated farming installation with one product, if the work surface capacity to receive product is higher than the load capacity of the mobile workstation, the loading action is split into several sub-actions of loading. Each of these sub-actions are performed on a different portion of the work surface. These sub-actions can be also repeated on the same portion of the work surface to increase the thickness of the product by adding a layer of product over previous layer(s) of product already loaded.

According to different aspects, it is possible to provide the one and / or the other of the characteristics below taken alone or in combination.

According to one embodiment, the mobile workstation further comprises at least one sensor able to measure at least one parameter of the substrate or of air surrounding the substrate, and in that the mobile workstation comprises at least one active system comprising a resource release system adapted to release at least one vital resource upon activation based on monitoring of the at least one parameter.

Thanks to these provisions, the workload of the operator is reduced: actions previously performed by the operator do not need to be performed manually any more. The operator also does not need to go and directly analyze the substrate to collect the relevant parameters necessary to decide which action should be performed on the substrate. The parameters collected by the mobile workstation are transmitted either to the operator, or to the system to move the mobile workstation in order to be delivered respectively to the operator or to the displacement system. The energy brought and used by the mobile workstation is energy that the operator does no need to spend him/herself to perform actions onto the substrate. Resources, such as fluids, liquids, water, nutriment, vapor, air with appropriate rate of oxygen and/or carbonic gas, which are useful for the growth of the larvae, can be delivered by the mobile workstation in required quantities and directly where it is requested on or into the substrate or in the surrounding air.

According to one embodiment, one sensor is positioned on a portion of the mobile workstation adapted to move from above the substrate to inside said substrate.

Thus, the same sensor can be used either to measure one parameter of the air around the substrate, or the same parameter of the substrate. For instance, the temperature or the humidity of the air and of the substrate are both important parameters that thus can be measured by the same sensor. The sensor is positioned for example on one tool that can be positioned inside or outside the substrate, like a plough.

According to one embodiment, the displacement system comprises an engine and at least one driving wheel connected to said engine, and adapted for mechanical cooperation with said guiding system of the insect farming installation; said engine being adapted to rotate the driving wheel in both rotating directions, and the control system is adapted to deliver signals to activate the rotation of the driving wheel in both rotating directions.

Thus, the mobile workstation can be displaced over the whole container, backward and forward. It can also cover only one portion of the container if needed. Thanks to the at least two rails, the mobile workstation is stable and bears on the rails to perform some actions.

According to one embodiment, the mobile workstation comprises at least one active system comprising a substrate removal system able to remove the substrate, and the mobile workstation comprises an actuation system adapted to place the substrate removal system alternatively in an active condition where it is adapted to remove the substrate and a passive condition where it is adapted to stay outside of the substrate.

Thus, at the end of the step 2 of the farming cycle, the operator does not need to manually clean the container before starting a new step 2 farming cycle.

According to one embodiment, the mobile workstation comprises at least one active system comprising a humidification system.

Thus, the operator does not need to manually humidify the substrate.

According to one embodiment, the mobile workstation comprises at least one active system comprising a substrate displacement system adapted to move the substrate, and the mobile workstation comprises an actuation system adapted to place the substrate displacement system alternatively in an active condition where it is adapted to move the substrate and a passive condition where it is adapted to stay outside of the substrate.

Thus, the operator does not need to manually put in place the substrate at the beginning of the step 2 of the farming cycle, to manually equalize the substrate height, and to manually remove the substrate at the end of the step 2 of the farming cycle.

According to one embodiment, the mobile workstation comprises an energy reserve stock adapted to provide energy to move the mobile workstation and/or an actuation system.

Thus, in case the external source of energy is stopped for any reason, the displacement system can still perform an emergency procedure to put the mobile workstation in an active mode and to move it back to the standby position.

According to one embodiment, the mobile workstation comprises at least one active system comprising a brush adapted to clean a substrate-receiving container of the farming installation, and an actuation system adapted to place the brush alternatively in an active condition where it is adapted to clean the container and a passive condition where it is adapted to stay outside of the container.

According to one aspect, the invention relates to an automated farming installation comprising a continuous profiled container adapted to receive substrate, the mobile workstation according to one of the claims 1 to 9, and a guiding system adapted to mechanically cooperate with the displacement system of the mobile workstation.

According to one embodiment, the automated farming installation further comprises a lift system adapted to vertically move the mobile workstation from one level comprising said continuous profiled container to another level comprising another continuous profiled container.

According to one embodiment, the lift system is adapted to move substrate from a substrate supply to a continuous profiled container.

### BRIEF DESCRIPTION OF THE DRAWINGS

Embodiments of the invention will be described below with reference to the drawings, described briefly below:
Fig. 1 is a schematic lateral view showing an example of an embodiment on an insect farming installation.
Fig. 2 is a front view of the insect farming installation.
Fig. 3 is a schematic lateral view of an example of a lift system.
Fig. 4 represents a top view of the mobile workstation in one working position.
Fig. 5 represents a side view of the mobile workstation in inactive position.
Fig. 6 represents a side view of the mobile workstation, with the ploughs in a standby position.
Fig. 7 represents a side view of the mobile with the ploughs and bucket in active position.

In the drawings, identical references designate identical or similar objects.

### DETAILED DESCRIPTION OF THE INVENTION

One example of a mobile workstation 10 dedicated to be used in a farming installation 1 will be described below.

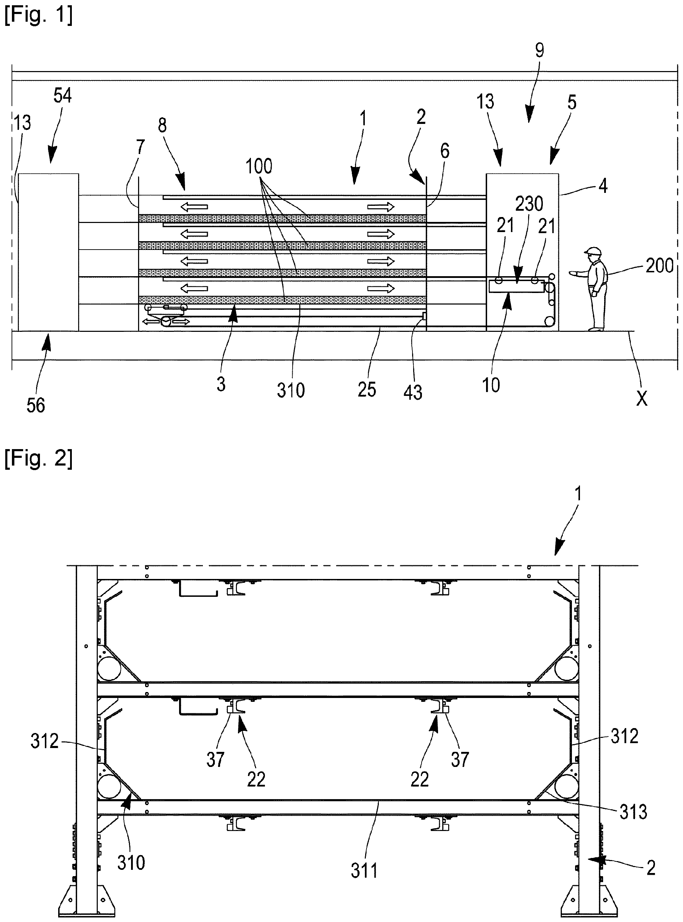

As can be seen on Figure 1, the farming installation 1 comprises a breeding module 8, an entry module 9 and an output module 54. The breeding module 8 comprises a structure 2. The structure 2 of the breeding module 8 of the farming installation 1 comprises two lateral vertical frames. Between the frames, horizontal containers 310 are fixed one above the other. The containers 310 extend along a longitudinal direction. The containers are profiled along the longitudinal direction. For example, as shown on Fig. 2, the containers 310 have a U-shaped cross-section transverse to the longitudinal direction. The containers 310 have a flat horizontal base 311 and two lateral vertical walls 312. The junction between the base 311 and the walls 312 may be at a right angle, or through one or more rounded or angled intermediate sections 313. The walls may not be exactly vertical, and may be defining a tapered profile tapering toward the base. This surface extends without discontinuity from one end 6 to the other end 7 along the longitudinal direction. At least two rails 22 are fixed above each container. Each rail 22 for example comprises a toothed rack 37 extending longitudinally along the rail 22. A mobile workstation 10 is moved along the rails 22 above the containers, in order to treat the containers or the substrate contained therein, which are later called a "work area" 3. A source 43 of energy, such as for example electrical energy and/or pneumatic energy, is also provided. The mobile workstation 10 is supplied by the source(s) of energy through suitable cables and hoses. The installation further comprises a central control (not shown). The farming installation 1 of this embodiment comprises an entrance longitudinal end 4, through which substrate is brought into the farming installation, and an opposed output end 13 through which substrate is removed from the farming installation.

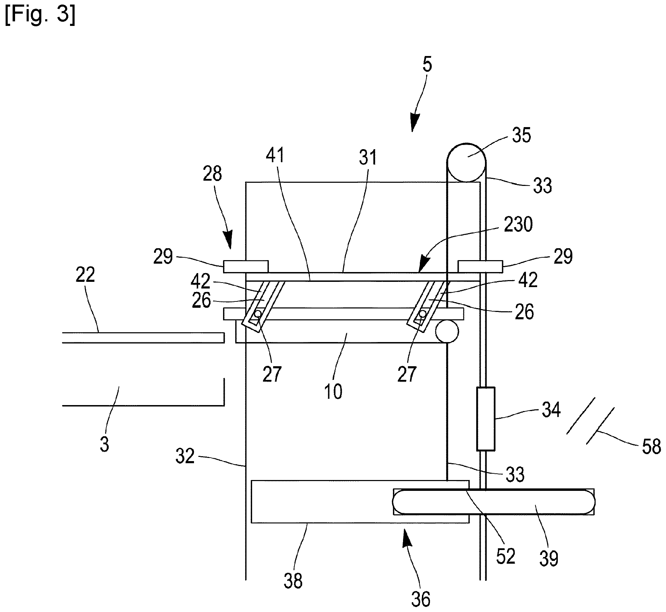

In one embodiment, to position the mobile workstation precisely at the entrance of each work area, as shown on **figure 1****,** the farming installation comprises a lift system 5. The lift system 5 is provided at a first longitudinal end of the farming installation 1. In one example, the lift system 5 is part of the entry module 9 of the installation, and facing the entrance end 6 of the breeding module 8. In this embodiment, as will be described below, the lift 5 may be used to bring the substrate into the containers. An example of a structure of the lift system 5 will be described thereafter in relation to figure 3. The lift system 5 comprises a static structure 32 and a vertically movable part 230 adapted to move vertically with respect to the static structure 32. As visible in Figure 3, the vertically movable part 230 is provided with a mobile workstation holder **41**, which is adapted to hold the mobile workstation 10 in two positions. The mobile workstation holder **41** comprises for example a main structure 31 such as a rectangular frame equipped with an engine (not shown) able to move the mobile workstation 10 with respect to this structure. For example, the movement of the mobile workstation 10 with respect to the main structure 31 is a translation movement along an axis which is inclined with respect to the vertical. The inclination axis is for example less than 45°. For example, four links 42 are integral with the rectangular frame, oriented in the same direction and with the same angle. The links have oblong holes 26 in the middle of each link, adapted to receive guided devices 27, such as wheels, linked to the mobile workstation 10. Thus, the engine can move the mobile workstation 10 up and down according to an oblique direction, getting closer to the work area 3 in the lowest position. The mobile workstation holder 41 is vertically moved within the lift system 5 from a position one level above the lowest level of work area up to one level above the highest level of work area. During this vertical movement, the mobile workstation 10 is in the up position with respect to the mobile workstation holder 41, close to the rectangular frame.

The mobile workstation holder 41 further comprises a load relief system 28. The load relief system 28 may comprise one or more arms 29 which are provided movable with respect to a main structure 31 of the mobile workstation holder. One or more engines (not shown) may be provided to move the arms with respect to the main structure 31. The arms 29 may be moved with respect to the main structure 31 between an expanded position, as shown on Fig. 3, and a retracted position. In the retracted position, the arms 29 do not interfere with the static structure 32 of the lift system 5 upon vertical movement of the vertically movable part 230. In the expanded position, the arms 29 expand with respect to the main structure 31, so that they would interfere with the static structure 32 upon a vertical movement of the vertically movable part 230.

The mobile workstation holder 41 and the mobile workstation 10 move together vertically. When the mobile workstation holder 41 reaches a level associated to an intended work by the workstation 10, the arms 29 are moved to their extended position where they can interact with the static structure 32. For example, the arms 29 in this position rest on the static structure 32. The load of the mobile workstation holder 41 is thus supported directly by the static structure 32. Thus, the group of the mobile workstation holder 41 and the mobile workstation 10 are stabilized in a fixed vertical position configuration. Once in a fixed vertical position configuration, the mobile workstation 10 is moved with respect to the mobile workstation holder 41. The mobile workstation 10 is moved down along the links 42 until it is facing the guide rail 22. Fig. 3 shows an intermediate position. A guiding system may be provided to precisely align the mobile workstation 10 with the rail 22, so as to cope with the low precision of the positioning by the lift system. This guiding system may for example be based on male cones positioned ahead of the mobile workstation holder 41 which are blocked by female cones (not illustrated) at one entrance edge of each work area. Thus, the mobile workstation 10 may be stopped before it reaches the end of its movement track with respect to the mobile workstation holder 41. Thus, the suspension wheels 21 of the mobile workstation 10 are precisely aligned with the rails 22 of each work area.

The mobile workstation 10 can thus be controlled to be moved along the rails 22, whereby it is disassembled from the vertically movable part 230.

When the mobile workstation 10 finishes working at this work area, it is assembled to the lift system 5 according to a reverse order of the above steps. The mobile workstation 10 is first moved back to cooperate with the vertically movable part 230. The mobile workstation 10 is moved up toward the main structure 31, and the arms 29 are moved to their retracted position, so that the vertically movable part 230 can be moved to another level. It should be noted that, according to this embodiment, it is not compulsory to use the load relief system 28. This embodiment may alternatively be used with the vertically movable part 230 suspended in the lift.

According to one embodiment, the lift system is built as described below. The vertically movable part 230 is assembled to a lifting cable 33. A counterweight 34 movable vertically is also assembled to the lifting cable. When the vertically movable part 230 is on the topmost possible position, which is one level above the highest work area, the counterweight 34 is still suspended thanks to a set of pulleys 35. Thus, the balance ensured by the counterweight is ensured whatever the vertical position of the top frame is. The lift system engine can move the cable.

According to one embodiment, the lift system 5 is also used to supply with substrate material. In this embodiment, the lift system 5 comprises a second vertically movable part 36. The second vertically movable part 36 is movable vertically within the static structure. For example, the second vertically movable part 36 is below the first vertically movable part 230 and will remain below the vertically movable part 230 throughout operation of the system.

The lift system 5 comprises an engine to move the second vertically movable part 36. According to one example, the same engine is used to either move the first vertically movable part 230 or the second vertically movable part 36. According to this embodiment, the lift system 5 is built as described on Fig. 3. The second vertically movable part 36 is assembled to the lifting cable 33. The lift system 5 comprises a control switch adapted to control the assembly of the first vertically movable part with the second vertically movable part. In a first configuration, the first vertically movable part is assembled to the second movable vertical part, so that the two parts may be moved vertically together. In particular, when, as described above, the first vertically movable part 230 is supported directly by the static structure 32 of the lift system 5, it is possible to disassemble the second vertically movable part 36 from the first vertically movable part. The second vertically movable part 36 may be used to move substrate vertically in the lift system, in order for the substrate to be distributed to the work area 3.

By default, the position of the second vertically movable part 36 is at the bottom of the lift system 5 and the first vertically movable part 230 is assembled to it and located just above it.

According to one embodiment, as shown, the second vertically movable part 36 comprises a base structure 38 and a mobile carrier 39 adapted to move with respect to the base structure 38. In particular, as shown, the mobile carrier 39 may be controlled to move horizontally with respect to the base structure, between a compact position, where the second vertically movable part 36 can be moved vertically, and an expanded position, where the mobile carrier 39 expands partly outside of the static structure 32 of the lift system, thereby preventing the second vertically movable part 36 from being moved vertically. In particular, in the expanded position, the mobile carrier 39 may be placed under a substrate supply system 58. For example, this movement is allowed only at one level of the lift system, for example, the bottommost level. For example, the expanded position is at the rear of the lift system 5, i.e. on the side of the lift system opposite to the work areas. According to yet another embodiment, the mobile carrier 39 may also take another expanded position, where part of the mobile carrier 39 extends above a work area. This eases the transfer of substrate from the mobile carrier 39 to the work area 3 by the mobile workstation 10. For example, this other expanded position may be reached only at the levels of the lift system corresponding to a work area, but not at the bottommost level. Further, the mobile carrier 39 may comprise a base and conveyor belt 52 which runs cyclically with respect to the base.

The mobile workstation 10 is able to move horizontally over the flat surface of the containers 310 of the installation 1, and also vertically thanks to the lift system 5 installed on one end of the installation 1, as described above. The motion of the mobile workstation 10 is defined as "forward" when the mobile workstation 10 moves in the direction from the lift system 5 toward the output module 54, and as "backward" when the mobile workstation moves in the direction from the output module 54 toward the lift system 5.

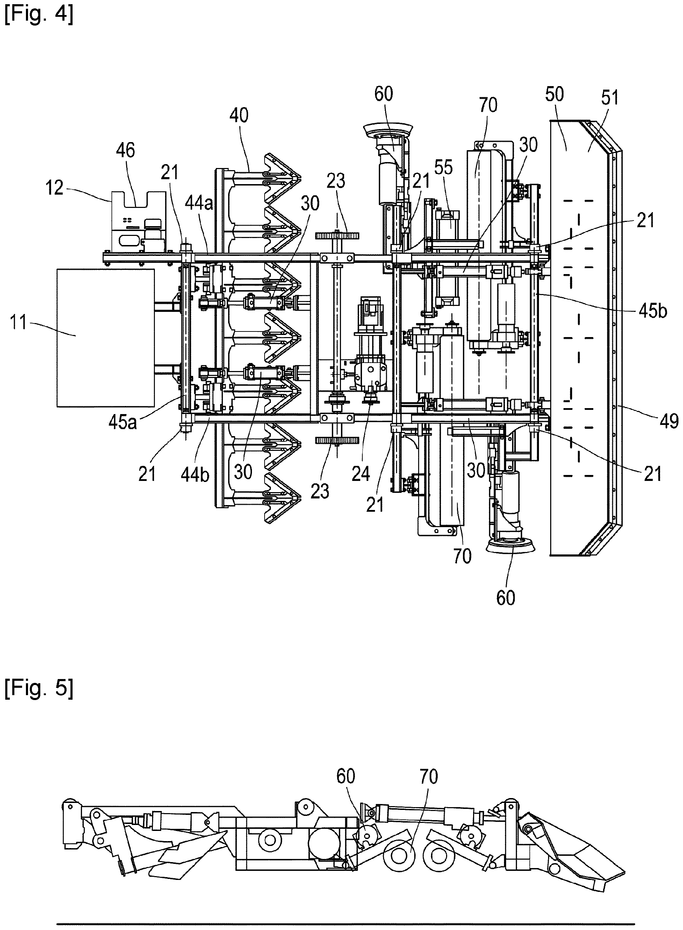

As shown on Figures 4 and 5, the mobile workstation 10 comprises a structure 20 carrying the various other functional components of the mobile workstation 10. The structure 20 for example comprises a rigid frame, but may alternatively comprise other mechanical structural elements such as plates. The frame for example comprises two side longitudinal parallel beams 44a, 44b assembled to one another through cross-beams 45a, 45b. There might be more than two cross beams. The frame carries a connection system 12 adapted to connect the workstation 10 to one or more cables. For example, the frame carries an electronic connection device 46 comprising the connection system 12. The cables comprise an electrical cable with power, signal lines such as electrical or optical signal lines, and/or a compressed air line, one or more liquid lines, etc... The connection system 12 is provided at the rear of the frame, and the cables extend backward from the mobile workstation 10. A guiding chain carrier system 25 is connected to the mobile workstation 10 at one lateral side of the mobile workstation. Thus, the guiding chain carrier system 25 can be deployed or retracted while the mobile working station 10 is moving in the backward or the forward direction.

The frame carries the control system of the mobile workstation. The control system comprises a controller, for example a computer 11 able to deliver control signals to other components of the mobile workstation, for example able to control the pneumatic systems. The control system is provided close to the connection system, for example at the rear of the mobile workstation 10.

The frame carries at least three suspension wheels 21 unaligned and defining one horizontal moving plane. The frame further carries at least one driving wheel 23.

The suspension wheels 21 are able to be placed and to run on the rails 22. In our embodiment, six suspension wheels 21 are separated into two groups of three aligned wheels. The size and lateral spacing of the wheels are adapted to the two rails. The at least one driving wheel 23 is connected to an engine 24 to rotate the driving wheel 23 in both rotating directions. The driving wheel 23 has a toothed surface able to interact with the fixed linear toothed rack 37 running longitudinally. The interaction between the rack 37 and the toothed surface of the wheel 23 allows a precise positioning of the mobile workstation.

In the present embodiment, two driving wheels 23 are positioned on the lateral sides of the mobile workstation, along a horizontal line perpendicular to the movement direction and crossing the centre of gravity of the mobile workstation 10. Thus, the stresses related to the motion of the mobile workstation 10 at the level of the suspension wheels 21 are minimized.

The driving wheel 23 can move the mobile workstation in two opposite "forward" and "backward" directions. The controller is able to receive a movement command from the central control and to deliver signals to activate the supply of energy to the engine, hence causing rotation of the driving wheel 23 in either rotating direction.

The alimentation source of the engine 24 is for example electricity provided through the cable supported by the guiding chain carrier system 25, from the source 43 of electricity to the mobile workstation 10.

The engine 24 and driving wheels 23, as well as the transmission of movement from the engine 24 to the driving wheels, form an example of a displacement system to move the mobile workstation.

The mobile workstation 10 comprises tools able to perform one action onto the substrate. The mobile workstation 10 further comprises actuators arranged to move respective tools from an upper stand-by position to a lower active position.

Actuators can be cylinders 30 activated by different means: compressed air, electricity, or hydraulic power. In this embodiment, the cylinders 30 are powered by compressed air. The compressed air is provided through the compressed air pipe supported by the guiding chain carrier system 25, from the source 43 of compressed air to the mobile workstation 10. The mobile workstation 10 may bear a compressed air reserve stock 55. The air reserve stock 55 is designed to provide compressed air at a higher pressure than can be provided from the source 43. The air reserve stock 55 may be used to provide energy to the cylinders 30 upon need, and will be filled from the source 43 when partially or totally depleted.

Some tools of the mobile workstation 10 are passive tools: being in the lower active position, combined with the movement of the mobile workstation 10 in its longitudinal direction, action of the tool is applied on the substrate.

Some tools are active tools: in addition to a passive tool action mode, the active tools can be moved in action mode by an engine to give them, for example, a rotation movement. Thus, the efficiency of the tool is increased.

For example, the passive tools are ploughs 40 and a pusher 50, and the active tools are brushes 60, 70.

In our embodiment, there are four brushes of two different types: two lateral brushes 60 and two bottom brushes 70. The lateral brushes 60 are designed to brush the lateral walls of the container. The bottom brushes 70 are designed to brush the bottom of the container. Each brush can be moved by a dedicated electric engine. Brushes 60, 70 can rotate alternatively in the two opposite rotation directions for a better brushing efficiency.

In the upper standby position, as shown on Fig. 5, the brushes 60 and 70 are out of the substrate. In the lower active position (not shown), the brushes 60, 70 are in contact with the internal surface of the container, such as the internal surface 47 of the bottom plate of the container. The combination of the brushes 60, 70 in the lower position during movement of the workstation 10 covers all the surface of the container 310 which was in contact with the substrate.

In the present example, the brushes are provided in the front half of the workstation. The bottom brushes 70 are not aligned with one another, but are disposed longitudinally offset from one another. In the upper standby position, the lateral brushes 60 are disposed longitudinally offset from one another. Hence, the brushes are provided in an asymmetric arrangement, which enables to reduce bulk of the mobile workstation in the standby configuration of the brushes.

Brushes 60, 70 can be easily mounted and unmounted with simple tools with respect to the structure 20 of the workstation, enabling them to be replaced in case of brush wear.

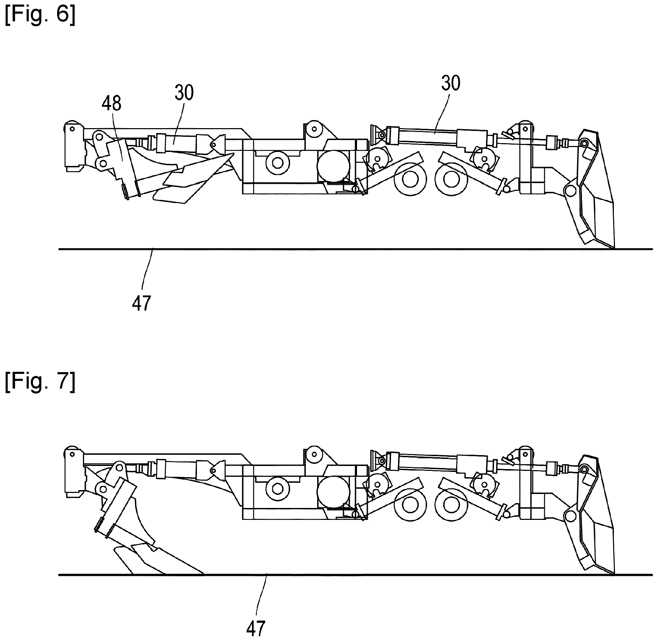

To cover the width of the container, ploughs 40, for example seven aligned ploughs 40, are mounted on a movable stem 48. The ploughs can be moved vertically from their upper standby position (Fig. 5) to their lower active position (Fig. 7) by compressed air cylinders 30.

The ploughs 40 can be oriented either in the forward direction, or the backward direction, or in both directions. Ideally, in both directions, the ploughs 40 of the backward direction are not aligned with the ploughs 40 of the forward direction. Thus, the substrate is ploughed along different lines, enhancing the ploughing effect.

In the presented embodiment, ploughs 40 are oriented in the forward direction.

Ploughing prevents crusting on the surface of the substrate, and enhances gas exchange with the outside.

In the present example, the ploughs 40 are provided in the rear half of the workstation.

The pusher 50 is at the front of the mobile workstation 10. The pusher 50 comprises a sensibly flat plate 51. Hence, the pusher does not comprise a hollow bucket which would be able to contain a large quantity of substrate. The size of the pusher 50 is adapted to match the container 310: The length and height of the pusher 50 are adapted to cover the transversal section of the container up to the level of the substrate. The pusher 50 is able to be oriented with respect to a horizontal axis perpendicular to the direction of movement of the mobile workstation. The orientation of the pusher 50 with respect to this axis may be modified by actuators.

The pusher 50 can be positioned in an upper position, wherein the pusher 50 is above the surface of the substrate (Fig. 5), or in a lower position wherein the bottom of the pusher 50 can touch the internal surface 47 of the bottom of the container 310. This lower position is seen on Figs. 6 and 7. The pusher can also be positioned in an intermediate position between the upper and the lower one.

The front edge 49 of the pusher has both a sharp shape in order to shear the substrate when moving from the upper position to the lower position. The front edge of the pusher may comprise a metallic body covered with an elastomeric coating in order to seal the bottom of the pusher 50 to the bottom of the container in the lower position. The actuators to move the pusher 50 between the upper and the lower positions are for example two compressed air cylinders 30. The power provided by the actuators has to be enough to enable the pusher to cut through the substrate.

Thus, in the lower position as shown on Figs. 6 and 7, the pusher 50 can separate a portion of the substrate (to the right of the pusher 50) from the bulk of substrate (to the left of the pusher 50), and push it outside of the container. In fact, the container 310 can be long of several meters, and contain substrate over a given thickness, 15 cm of substrate thickness for example. In that case, pushing in and out all the substrate of one container requires such an energy that the structure of the mobile workstation is not able to withstand the corresponding stresses. So, the substrate is installed in the container, and removed from the container, portion of substrate by portion of substrate. Each portion is about 230 kg of substrate.

To push one portion of the substrate out of the container, the pusher 50 cuts through the substrate to its lower position, vertically oriented, as shown on Figs. 6 and 7. The portion of substrate to be removed from the container is in front of the pusher 50, and the mobile workstation 10 moves forward toward the output end.

To push one portion of the substrate into the container, the pusher 50 is in the lower position, vertically oriented, the portion of substrate to be positioned into the container is in front of the pusher 50 at an entry position of the container, the mobile workstation 10 moves toward the position where the portion of substrate has to be placed.

The system which was described above may be operated as follows.

At the beginning of one second aging phase, the operator **200** launches the sub phase of loading substrate **100.**

Initially, the mobile workstation **10** is at a standby position, vertically at a given level, for example at the top level, and on the lift system **5.** The mobile carrier 39 is extended below the supply system 58. The supply system **58** is for example provided so as to provide substrate from the side of the lift system opposite to the side facing the work area **3.** Substrate **100** is loaded on the conveyor belt 52, until it reaches its loading limitation (step 1). In this embodiment, the capacity is limited to a part of the working surface, for example the equivalent of one meter long of substrate **100** of one work area **3.** One load of substrate is for example about 20-300 kg. During the loading of substrate, the conveyor belt 52 is activated, and moves with respect to the base, so that the whole surface of the conveyor belt 52 is provided with substrate, by providing substrate at a static location, and moving the conveyor belt so that an empty portion of the conveyor belt is in the static location and receives the substrate. The mobile carrier 39 is then moved within the static structure 32 of the lift system 5. The second vertically movable part is moved upward until it faces the container to be filled.

Then, the substrate is supplied to the part of the container closest to the mobile workstation **10.** For example, the conveyor belt 52 is activated one more time, so that substrate accumulated on the conveyor belt is poured into the container at the entry side. Then, the second vertically movable part 36 is commanded to move back below the supply 58 in order to receive a new load of substrate.

The mobile workstation 10 is moved down along the links 42, and the pusher 50 is actuated to be placed in its pushing configuration.

Then, the controller launches the first mobile workstation **10** movement process: normal horizontal process until reaching the opposite end of the working area **3** minus one portion, for example one meter (step 2).

When the substrate **100** is loaded over the appropriate length (for example one meter, which is one exemplary distance) of the work area 3, the tools are stopped by the controller, and the normal horizontal process is completed by the return of the mobile workstation to the standby position (step 3). If necessary, before doing that, the pusher 50 is moved to its upper passive position, so as not to contact the container 3 during the return movement.

Steps 1 to 3 are repeated. At each occurrence, the horizontal normal process at step 2 is reduced by the previously filled length of the working area until the dedicated work area **3** is completely filled with substrate **100.**

Then a second movement process is performed: the normal vertical process (step 4) in the upward direction until the mobile workstation reaches another level, for example the highest level of the working area 3. Then, the steps 1 to 3 are repeatedly performed at that level in order to fill the container at that level, then the normal vertical process in the downward direction is launched by the controller, and the above process is repeated at each level until the mobile workstation 10 is back to its standby position (step 5).

Once the substrate **100** is in place on all the work areas **3,** some steps of treating the substrate can be repeated as needed during the 7 days of the cycle. These actions are performed by activating different actuators of the mobile workstation **10.** These actions may be launched based on a predetermined schedule or feedback from sensors. For example, one action is to plough the substrate. When a ploughing operation is decided, the ploughs are moved to their lower active position. Forward movement of the mobile workstation 10 will then cause the ploughs to shear through the substrate, promoting air exchanges and uncrusting the surface. After the ploughing operation is complete, the ploughs are moved up to their upper passive position.

Another action is to provide fluids to the substrate, as will be detailed below.

Another action is to perform measurements, in or close to the substrate, as will be described below.

Some of these actions may be performed simultaneously.

The second aging phase usually lasts 7 to 8 days. When the operator decides that the larvae (i.e. BSF) have reached maturity, he launches the substrate removal process. For example, the mobile workstation 10 is at a standby position, vertically at the lowest level, and on the lift system **5.**

Then, the controller launches the first movement process: normal horizontal process until reaching the work area **3** without substrate **100** minus one meter (step 2').

The first occurrence of step 2' on one given level is reaching the opposite end of one work area **3** minus one meter.

Then the pusher 50 is activated by the controller (step 2"). This function is ensured by the action of pressurized air at the level of the pusher. The pusher is hence moved slicing through the substrate to its bottom position. Then, the normal horizontal process is applied to move along the remaining surface of the container until reaching the opposite side edge of the container, pushing one meter long of substrate **100** out of the work area 3 to a reception area 56, for example into a collecting bin of the output module 54 (step 2‴). Then, the pusher 50 is moved to its upper passive position, and the normal horizontal process is launched in the opposite direction until reaching a position of the work area 3 corresponding to a length of substrate to be unloaded (step 3').

Steps 2', 2", 2‴ followed by 3' are repeated until the lowest work area **3** is empty (cycle 1).

Then the second movement process is applied by the controller: the normal vertical process (step 5') in the upward direction until reaching the highest level of the work area 3.

Then, the steps 2', 2", 2"' and 3' are repeated until the work area **3** is empty (cycle 1).

Then, the normal vertical process in the downward direction is launched by the controller until the mobile workstation reaches the next level (step 5').

Then, cycle 1 followed by step 5' is repeated until this work area **3** is empty.

Thus, all the substrate **100** is pushed out of the work surface **3.** This is the end of one second aging phase cycle. As soon as one second aging phase cycle is achieved, another second aging phase cycle can be launched.

A cleaning phase between two second aging phase cycles can be added. For example, the mobile workstation is moved at one end of an empty container, and the brushes 60, 70 are moved in their down active position. Brushes are activated, and the mobile workstation 10 is moved forward. Cleaning fluid, such as water or water-based liquid, may also be brought to the container to be cleaned at that time, as described below. The mobile workstation is moved forward with the brushes in operation, so as to clean the container. The output module 54 may comprise a specific frass receptacle, in order to receive the frass pushed through the exit by the brushes, so that the frass is not mixed with the previously collected substrate. Then, the brushes are stopped and moved to their upper passive position.

Another function of the pusher 50 is to level the substrate. The pusher 50 is set in the upper position, and the orientation of the pusher 50 is set up in order that a portion of the pusher 50 is at the horizontal level of the desired level of substrate. This position of the pusher 50 is the equalizer position. When the pusher 50 is in the equalizer position, when the mobile workstation 10 is moving, the substrate level is equalized by the pusher 50. Further, the height of the equalizer position may vary during the rearing of the insects. Hence, the position and orientation of the pusher 50 in equalizer position may be different depending on the age of the substrate.

The mobile workstation 10 comprises at least one sensor able to measure at least one relevant parameter regarding the status of the substrate or the status of the air.

The information of the sensors can be sent through a cable to the computer 11 of the mobile workstation and also through another cable to the remote computerized unit.

The sensors collect for example the temperature, the humidity, the level of oxygen in the air, the pH level of the substrate, etc. Two groups of sensors can be provided on the mobile workstation 10: the environmental sensors to measure parameters of the environment of the substrate, and the substrate sensors to measure parameters of the substrate.

Environmental sensors can be mounted on the structure 20 of the mobile workstation to measure parameters of the air nearby the substrate but outside the substrate.

Substrate sensors can be mounted on a stem part of the mobile workstation and extended below the frame of the mobile workstation to remain in the substrate when the mobile workstation is moving. The shape of the sensor stem is designed to reduce the hindrance to the movement.

The sensor stem can also have an upper position and a lower position, according to the same principle as the tools described here above. Thus, the same sensor can be used alternatively as a substrate sensor or an environmental sensor.

The environmental sensors may be positioned between 1 and 50 cm above the surface of the substrate.

The mobile workstation 10 is alimented by at least one vital resource for the development of living organisms, the vital resource being released by one dedicated system of the mobile workstation 10.

A humidification system is one vital resource for the development of living organisms that can be installed on the mobile workstation.

The water is provided through a water pipe supported by the guiding chain carrier system 25 from the source 43 of water to the mobile workstation 10. The guiding chain carrier system 25 avoids the water pipe from being crushed or pinched. The source of water can supply water at different temperatures between 1 and 40 degrees Celsius. Water may also be suppled mixed with organic nutriments. The pH of the supplied water can also be adjusted, which may in turn influence the pH of the substrate.

The water pipe is connected to one side of a valve that can be remotely controlled. On the other side of the valve, a network of drilled pipes is fixed on the mobile workstation 10.

Thus, the substrate can be humidified. The substrate can also receive organic nutriments during the second aging phase of the farming cycle. The temperature of the substrate can also be regulated thanks to the humidification system.

Hence, the guiding chain carrier system 25 may carry a plurality of fluid lines, comprising a plurality of liquid lines, including a fresh water line, a line of water and nutriment, and/or a washing fluid line.

A controller may control the release of one vital resource, such as air, water and/or nutrients, based on a monitoring of the above parameters measured by the sensors.

While exemplary embodiment of the invention has been described with reference to one main embodiment, it will be understood by those skilled in the art that various changes, omissions and/or additions may be made, and equivalents may be substituted for elements thereof without departing from the spirit and scope of the invention. In addition, many modifications may be made to adapt a particular situation or material to the teachings of the invention without departing from the scope thereof. Therefore, it is intended that the invention is not limited to the particular embodiment disclosed as the best mode contemplated for carrying out this invention, but that the invention will include all embodiments falling within the scope of the appended claims. Moreover, unless specifically stated any use of the terms first, second, etc. do not denote any order or importance, but rather the terms first, second, etc. are used to distinguish one element from another.

### NOMENCLATURE

1: farming installation
2: structure of the farming installation
3: work area
4: Entrance longitudinal end
5: lift system
6, 7: ends
8: breeding module
9: Entry module
10: mobile workstation
11: computer
12: connection system
13: output end
20: structure
21: suspension wheels
22: rails
23: driving wheel
24: engine
25: guiding chain carrier system
26: oblong holes
27: guided devices
28: load relief system
29: arms
30: cylinders
31: main structure
32: static structure
33: lifting cable
34: counterweight
35: pulleys
36: second vertically movable part
37: toothed rack
38: base structure
39: mobile carrier
40: ploughs
41: mobile workstation holder
42: links
43: source
44a, 44b: longitudinal beams
45a, 45b: Cross-beams
46: Electronic connection device
47: internal surface
48: Stem
49: Front edge
50: pusher
51: flat plate
52: conveyor belt
54: output module
55: reserve stock
56: reception area
58: supply system
60: lateral brush
70: longitudinal brush
100: substrate
200: operator
230: vertically movable part
310: container
311: flat horizontal base
312: vertical wall
313: intermediate section

## Claims

1. Mobile workstation for automated insect farming installation, comprising a displacement system to move the mobile workstation by mechanical cooperation with a guiding system of the insect farming installation, a control system adapted to control the mobile workstation, said mobile workstation being alimented by at least one source of energy and comprising at least one active system adapted to perform one action onto a substrate, wherein the active system comprises a ploughing system, **characterized in that** the mobile workstation comprises an actuation system adapted to place the ploughing system alternatively in an active condition where it is adapted to plough the substrate and a passive condition where it is adapted to stay outside of the substrate.

2. Mobile workstation according to claim 1, further comprising at least one sensor able to measure at least one parameter of the substrate or of air surrounding the substrate, and in that the mobile workstation comprises at least one active system comprising a resource release system adapted to release at least one vital resource upon activation based on monitoring of the at least one parameter.

3. Mobile workstation according to claim 2, wherein one sensor is positioned on a portion of the mobile workstation adapted to move from above the substrate to inside said substrate.

4. Mobile workstation according to any of claims 1 to 3, wherein the displacement system comprises an engine (24) and at least one driving wheel (23) connected to said engine, and adapted for mechanical cooperation with said guiding system of the insect farming installation; said engine being adapted to rotate the driving wheel in both rotating directions, and the control system is adapted to deliver signals to activate the rotation of the driving wheel in both rotating directions.

5. Mobile workstation according to one of the claims 1 to 4, wherein the mobile workstation comprises at least one active system comprising a substrate removal system able to remove the substrate, and the mobile workstation comprises an actuation system adapted to place the substrate removal system alternatively in an active condition where it is adapted to remove the substrate and a passive condition where it is adapted to stay outside of the substrate.

6. Mobile workstation according to one of claims 1 to 5, wherein the mobile workstation comprises at least one active system comprising a humidification system.

7. Mobile workstation according to one of claims 1 to 6, wherein the mobile workstation comprises at least one active system comprising a substrate displacement system adapted to move the substrate, and the mobile workstation comprises an actuation system adapted to place the substrate displacement system alternatively in an active condition where it is adapted to move the substrate and a passive condition where it is adapted to stay outside of the substrate.

8. Mobile workstation according to one of claims 1 to 7, wherein the mobile workstation comprises an energy reserve stock (55) adapted to provide energy to move the mobile workstation and/or an actuation system.

9. Mobile workstation according to one of claims 1 to 8, wherein the mobile workstation comprises at least one active system comprising a brush (60, 70) adapted to clean a substrate-receiving container of the farming installation, and an actuation system adapted to place the brush alternatively in an active condition where it is adapted to clean the container and a passive condition where it is adapted to stay outside of the container.

10. Automated farming installation comprising a continuous profiled container adapted to receive substrate, the mobile workstation according to one of the claims 1 to 9, and a guiding system adapted to mechanically cooperate with the displacement system of the mobile workstation.

11. Automated farming installation according to claim 10, further comprising a lift system adapted to vertically move the mobile workstation from one level comprising said continuous profiled container to another level comprising another continuous profiled container.

12. Automated farming installation according to claim 11, wherein the lift system is adapted to move substrate from a substrate supply to a continuous profiled container.
